(19) Europäisches Patentamt
European Patent Office
Office européen des brevets

(11) Publication number: **0 161 985**
**B1**

(12) **EUROPEAN PATENT SPECIFICATION**

(45) Date of publication of patent specification: **10.08.88**

(51) Int. Cl.[4]: **C 07 J 1/00,** A 61 K 31/565

(21) Application number: **85400868.7**

(22) Date of filing: **06.05.85**

(54) **17-Alpha-(aryloxy or alkoxy) androstenes.**

(30) Priority: **07.05.84 US 607919**

(43) Date of publication of application:
**21.11.85 Bulletin 85/47**

(45) Publication of the grant of the patent:
**10.08.88 Bulletin 88/32**

(84) Designated Contracting States:
**BE CH DE FR GB IT LI LU NL SE**

(56) References cited:

**JOURNAL OF MEDICINAL CHEMISTRY, vol. 18, no. 2, February 1975, pages 165-168, Washington, DC, US; A.J. SOLO et al.: "Synthesis and biological activity of some ethers of testosterone. Implications concerning the biological activity of esters of tetosterone"**

(73) Proprietor: **E.R. Squibb & Sons, Inc.**
**Lawrenceville-Princeton Road**
**Princeton, N.J. 08540 (US)**

(72) Inventor: **Haugwitz, Rudiger D.**
**16 River Drive**
**Titusville New Jersey (US)**

(74) Representative: **Descourtieux, Philippe et al**
**CABINET BEAU de LOMENIE 55 rue d'Amsterdam**
**F-75008 Paris (FR)**

**EP 0 161 985 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to new steroids having the formula

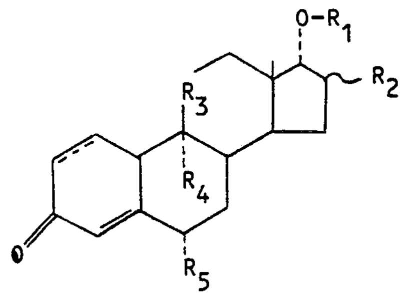

I

which have antiglucocorticoid activity. In formula I, and throughout the specification, the symbols are as defined below:

$R_1$ is aryl or alkyl;

$R_2$ is hydrogen, hydroxy, alkyl, alkoxy, aryloxy, oxo (=O), methylene (=$CH_2$), alkylthio, arylthio, alkanoyl, alkanoyloxy, or halogen;

$R_3$ is carbonyl or β-hydroxymethylene;

$R_4$ is hydrogen or halogen; and

$R_5$ is hydrogen, fluorine or methyl.

The dotted line in 1,2-position of the structural formulas herein represents the optional presence of ethylenic unsaturation.

The terms "alkyl" and "alkoxy" as used throughout the specification, refer to straight and branched chain alkyl groups having 1 to 10 carbon atoms.

The term "aryl", as used throughout the specification, refers to phenyl and phenyl substituted with 1, 2 or 3 alkyl, alkoxy or halogen atoms.

The term "halogen", as used throughout the specification, refers to fluorine, chlorine, bromine and iodine.

The steroids of this invention can be prepared utilizing as starting materials androstenes having the formula

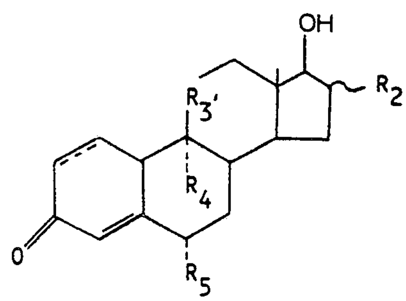

II

wherein $R_3'$ is carbonyl, β-hydroxymethylene or β-(acetyloxy)methylene.

Sequential reaction of a steroid of formula II with a phosphine such as triphenylphosphine, an azodicarboxylate such as diethylazodicarboxylate and a compound having the formula

$$R_1\text{—OH} \qquad \text{III}$$

yields the corresponding steroid having the formula

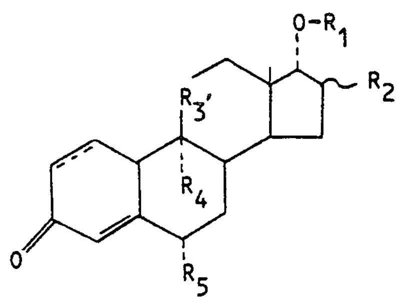

IV

If the product of formula IV is an 11β-(acetyloxy)androstene, deprotection can be accomplished using conventional techniques; *e.g.,* treatment of the protected steroid with an inorganic base.

The steroids of formula I have antiglucocorticoid activity and can be used in mammals to treat conditions caused by the presence of too high a level of administered or endogenous glucocorticoids. One such condition is Cushing's syndrome, and another is the retardation of growth in children. The steroids of this invention can be administered either orally or by injection, the dosage being adjusted for the relative potency of the particular steroid. The daily range will be between about 0.01 and 1 gram for a 70 kilogram mammal.

The following example is a specific embodiment of this invention.

Example 1
(11β,17α)-9-Fluoro-11-hydroxy-17-(phenoxy)androsta-1,4-dien-3-one

A) (11β,17α)-11-(Acetyloxy)-9-fluoro-17-(phenoxy)androsta-1,4-dien-3-one

(11β,17α)-11-(Acetyloxy)-9-fluoro-17-hydroxyandrosta-1,4-dien-3-one (365 mg, 1.0 mmole) and triphenylphosphine (288 mg, 1.1 mmole) were dissolved in 5.0 ml of dry tetrahydrofuran under a nitrogen atmosphere with magnetic stirring. Phenol (97 mg, 1.1 mmole) dissolved in 5.0 ml of tetrahydrofuran followed by diethylazodicarboxylate (.134 ml, 1.1 mmole) dissolved in 5.0 ml of tetrahydrofuran was added to the stirred solution. The mixture had a yellow color. After 67 hours, TLC (7:3 chloroform:ethyl acetate) showed little product formation. An additional 576 mg (2.2 mmole) of triphenylphosphine, 194 mg (2.2 mmole) of phenol, and 0.258 ml (2.2 mmole) of the diethylazodicarboxylate were added to the reaction mixture and it was refluxed. After 22.0 hours of reflux, TLC showed little starting material left. The mixture was added to cold water and extracted with chloroform. The pooled chloroform extracts were washed with water, dried over anhydrous magnesium sulfate and evaporated to an oil. This was preadsorbed on Baker silica gel (60—200 mesh) and flash-chromatographed on a column of silica gel (LP—1). The desired product was successfully eluted with a 2% ethyl acetate:98% chloroform mixture. The desired fractions were combined and evaporated *in vacuo* to yield a foamy solid which showed an NMR spectrum consistent with the expected structure.

B) (11β,17α)-9-Fluoro-11-hydroxy-17-(phenoxy)androsta-1,4-dien-3-one

(11β,17α)-11-(Acetyloxy)-9-fluoro-17-(phenoxy)androsta-1,4-dien-3-one was dissolved in tetrahydrofuran (12.0 ml) and methanol (6.0 ml) with magnetic stirring under a nitrogen atmosphere. A 12% sodium hydroxide solution (1.0 ml) was added and the mixture was stirred for 1.5 hours at room temperature before the addition of water and extraction with chloroform. The pooled extracts were washed with water, dried over anhydrous magnesium sulfate and evaporated to yield an oil. Two recrystallizations from a dichloromethane-petroleum ether mixture yielded 129 mg of an analytical specimen with consistent spectral data, melting point of 210°—212°C, $[\alpha]_D^{25}$ −13.7° (c, 1.0; chloroform).

*Anal.* Calc'd. for $C_{25}H_{29}O_3F_1$: C, 75.73; H, 7.37; F, 4.79.
Found: C, 75.76; H, 7.54; F, 4.76.

**Claims**

1. A steroid having the formula

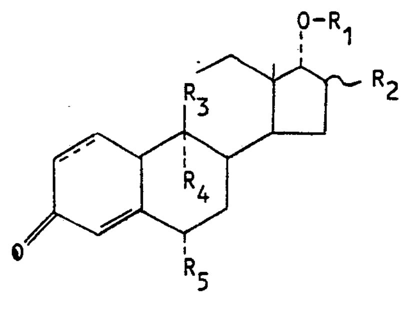

or 1,2-dehydro derivative thereof, wherein

$R_1$ is aryl or alkyl;

$R_2$ is hydrogen, hydroxy, alkyl, alkoxy, aryloxy, oxo, methylene, alkylthio, arylthio, alkanoyl, alkanoyloxy, or halogen;

$R_3$ is carbonyl or β-hydroxymethylene;

$R_4$ is hydrogen or halogen; and

$R_5$ is hydrogen, fluorine or methyl, wherein the term "aryl" refers to phenyl and phenyl substituted with 1, 2 or 3 alkyl, alkoxy or halogen atoms and the terms "alkyl" and "alkoxy" refer to straight and branched chain alkyl groups having 1 to 10 carbon atoms.

2. A steroid in accordance with claim 1 having the formula

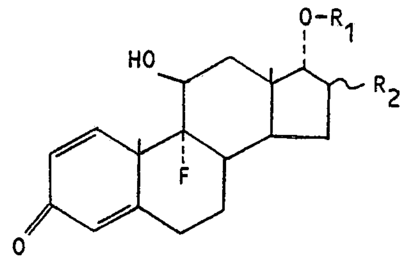

wherein $R_1$, $R_2$ and the terms "aryl", "alkyl" and "alkoxy" are as defined in claim 1.

3. A steroid in accordance with claim 1 or 2 wherein $R_2$ is hydrogen.

4. A steroid in accordance with claim 1, 2 or 3 wherein $R_1$ is aryl.

5. A steroid in accordance with claim 1, 2 or 3 wherein $R_1$ is alkyl.

6. A steroid in accordance with claim 1, (11β,17α)-9-fluoro-11-hydroxy-17-(phenoxy)androsta-1,4-dien-3-one.

**Patentansprüche**

1. Steroid der Formel

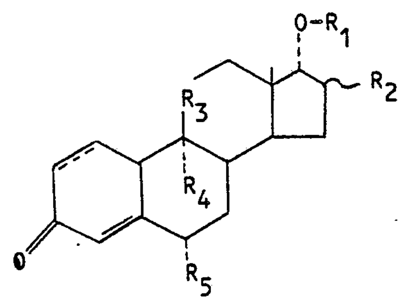

oder sein 1,2-Dehydroderivat, wobei bedeuten:

$R_1$ Aryl oder Alkyl;

$R_2$ Wasserstoff, Hydroxy, Alkyl, Alkoxy, Aryloxy, Oxo, Methylen, Alkylthio, Arylthio, Alkanoyl, Alkanoyloxy oder Halogen,

$R_3$ Carbonyl oder β-Hydroxymethylen,

$R_4$ Wasserstoff oder Halogen und

$R_5$ Wasserstoff, Fluor oder Methyl,

der Ausdruck "Aryl" Phenyl oder mit 1, 2 oder 3 Alkyl- oder Alkoxyresten oder Halogenatomen substituiertes Phenyl und

die Ausdrücke "Alkyl" und "Alkoxy" geradkettige und verzweigte Alkylgruppen mit 1 bis 10 Kohlenstoffatomen.

2. Steroid nach Anspruch 1 der Formel

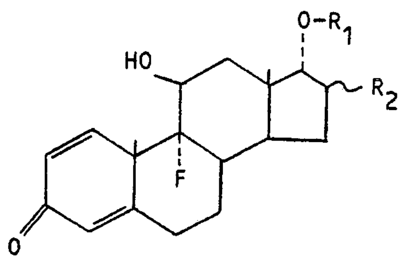

in der $R_1$, $R_2$ und die Ausdrücke "Aryl", "Alkyl" und "Alkoxy" wie in Anspruch 1 definiert sind.

3. Steroid nach Anspruch 1 oder 2, in dem $R_2$ Wasserstoff ist.

4. Steroid nach Anspruch 1, 2 oder 3, in dem $R_1$ Aryl ist.

5. Steroid nach Anspruch 1, 2 oder 3, in dem $R_1$ Alkyl ist.

6. Steroid nach Anspruch 1, (11β,17α)-9-Fluor-11-hydroxy-17-(phenoxy)androsta-1,4-dien-3-one.

**Revendications**

1. Un stéroïde répondant à la formule

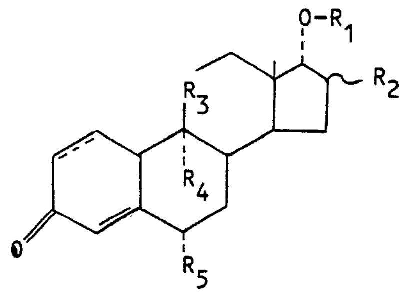

ou son dérivé 1,2-déshydro, dans lequel

$R_1$ est aryle ou alkyle;

$R_2$ est hydrogène, hydroxy, alkyle, alcoxy, aryloxy, oxo, méthylène, alkylthio, arylthio, alcanoyle, alcanoyloxy, ou un halogène;

$R_3$ est carbonyle ou β-hydroxyméthylène;

$R_4$ est l'hydrogène ou un halogène; et

$R_5$ est l'hydrogène, le fluor ou un groupe méthyle,

où le terme "aryle" désigne un groupe phényle et un groupe phényle substitué par 1, 2 ou 3 groupes alkyles ou alcoxy ou atomes d'halogènes et les termes "alkyles" et "alcoxy" concernent des groupes alkyles à chaîne droite ou ramifiée en $C_1$—$C_{10}$.

2. Un stéroïde selon la revendication 1 répondant à la formule

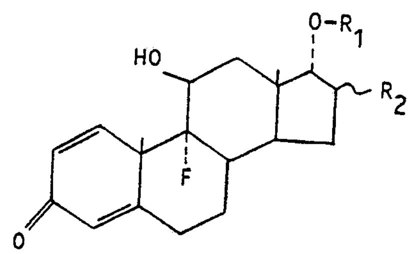

dans laquelle $R_1$, $R_2$ et les terms "aryle", "alkyle" et "alcoxy" sont tels que définis à la revendication 1.

3. Un stéroïde selon la revendication 1 ou 2, dans lequel $R_2$ est l'hydrogène.

4. Un stéroïde selon la revendication 1, 2 ou 3, dans lequel $R_1$ est un groupe aryle.

5. Un stéroïde selon la revendication 1, 2 ou 3, dans lequel $R_1$ est un groupe alkyle.

6. Un stéroïde selon la revendication 1, qui est la 9-fluoro-11β-hydroxy-17α-phénoxy-androsta-1,4-diène-3-one.